# EUROPEAN PATENT APPLICATION

(11) **EP 2 965 696 A1**
(43) Date of publication of application: **13.01.2016**
(21) Application number: 15425043.5
(22) Date of filing: 16.06.2015
(51) Int. Cl.: A61B 17/16, A61B 17/17, A61B 17/11, A61F 2/08, A61B 17/06, A61B 17/28, A61B 17/04

(54) **SET OF SURGICAL INSTRUMENTS FOR TENDON REPAIR**

(30) Priority: 08.07.2014 IT RM20140367
(71) Applicant: Unispinergy.com i.s.r.l., 00012 Guidonia Montecelio (Roma) (IT)
(72) Inventor: Giampiero, Monaco, 83011 Altavilla Irpina (Avellino) (IT)

(57) **Abstract**

This invention consists of a plurality of specific instruments, which are components of a novel surgical set of instruments, intended for repairing flexor tendons (100) of the hand. This surgical set of instruments is made of an high strenght endotendineous thread (1), a specific surgical needle (2), a specific holder (3) integrated with a coaxial drill (32), a contouring plier (4) with a drill piece (41) and relevant mechanism (42), a tapered mill cutter (51) with its guide wire (52), a specific tapered anchor (6) with optional accessories, as small tweezer (7) and pin (8), and, finally, some suitable threads for specific surgical core suture (91) and finishing suture (92). Using this surgical set, the surgeon gets each of the specific instruments he needs for best repair of a flexor tendon (100); basically, he has to pierce the bone on the back (203) of the phalanx (200) to create the hole (202) passing the high strenght endotendineous thread (1) before setting it and locking it by means of said tapered anchor (6).

This novel surgical set of instruments allows reinforcing the broken tendon very strongly, as it makes use of said tapered anchor (6) and of a suture made of core bites (91), merging the high strenght endotendineous thread (1) and the broken tendon ends (101, 102) in such a way that, as soon as this surgical tendon synthesis is completed, the relevant articulation (300) can be mobilized and loaded immediately in the frame of rehabilitation tasks

## Description

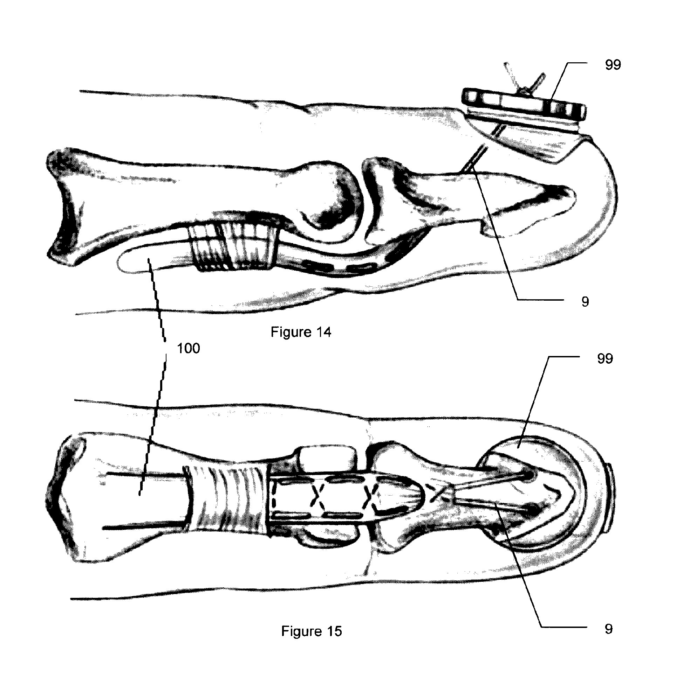

This invention consists of a surgical set of instruments to be used in the field of hand surgery for repairing flexor tendons. When the flexor tendon of a finger is detached from its distal insertion at the base of the phalanx, the surgeon can not simply perform a suture of the tendon [100], but - as shown in the figures 14-15-he must place a bite of suture [9] through the bone to reattach the tendon at its insertion place on the phalanx base. This state of the art technique is named "pull-out", because it uses a button [99] to tie the bite of suture [9] on the back of the nail, just outside the fingernail. However, this fastening system, based upon any generic set of surgical instruments, easily available, is affected by disadvantages well known, as the suture shows very poor tightness, such that the immobilization of the finger is required for at least one month, using cue and bandage. Moreover, pressure sores could arise due to the contact with the button [99].

The proposed invention consists of a novel surgical set of specific instruments, dedicated to the surgical repair of flexor tendons of the hand, solving these critical problems which affect the current state-of-the-art and achieving an exceptional tightness, unmatched yet in the flexor tendon repair art.

Indeed, as soon as this kind of tendon synthesis is completed, using said dedicated surgical set of instruments, the relevant articulation (300) can be mobilized and loaded immediately for rehabilitation tasks.

Early recovery of articular function is the most important and crucial advantage after the use of this invention, allowing the substantial reduction of the risks of postoperative complications compared with those of the previous art.

Said tendon repair surgical set of instruments consists, essentially, of an high strength endotendineous thread [1] adopted for tendon synthesis together with a set of further specific instruments, needed in the sequence of surgical phases, which are now listed in order of appearance : a specific surgical needle (2), a specific holder (3) integrated with a coaxial drill piece (32), a contouring plier (4) with a drill piece (41) and relevant mechanism (42), a tapered mill cutter (51) with its guide wire (52), a specific tapered anchor (6) with optional accessories as a small tweezer (7) and its locking pin (8) and, finally, some suitable threads for specific core suture (91) and for specific finishing suture (92).

More in detail, the initial section [11] of said high resistance thread [1] includes the coupling device [13] to the eye [21] of the specific surgical needle [2] .

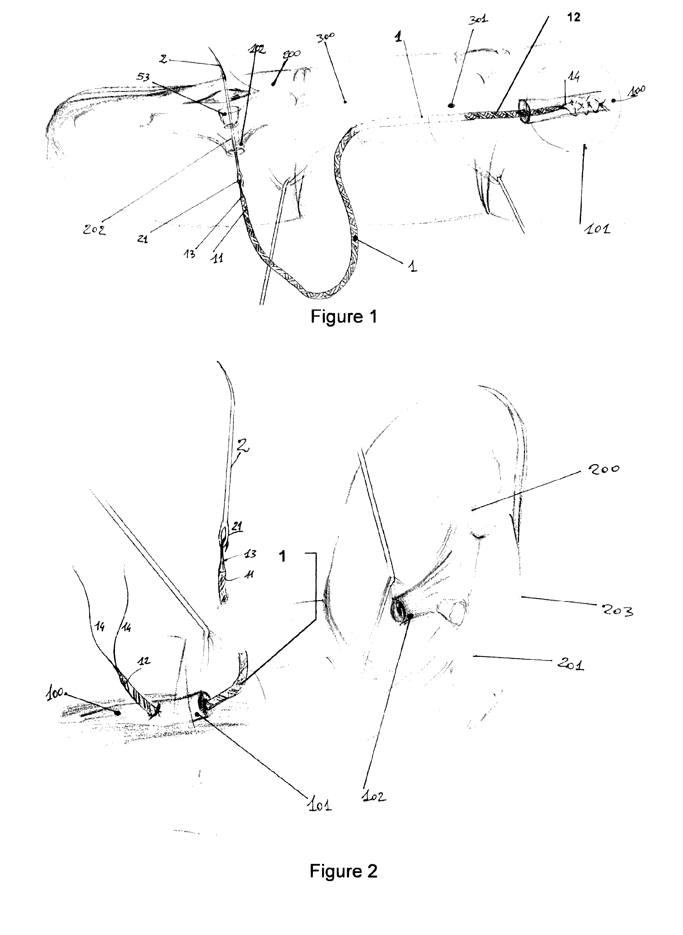

As shown in Figure 2, said thread [1] is inserted in the proximal lead [101] of the tendon to be repaired, it is dragged by the needle [2] and it's made to slide, until the final section [12] with thread ends [14] are set close to the proximal lead [101] of the broken tendon, in order to arm a strong suture, as depicted in Figure 1.

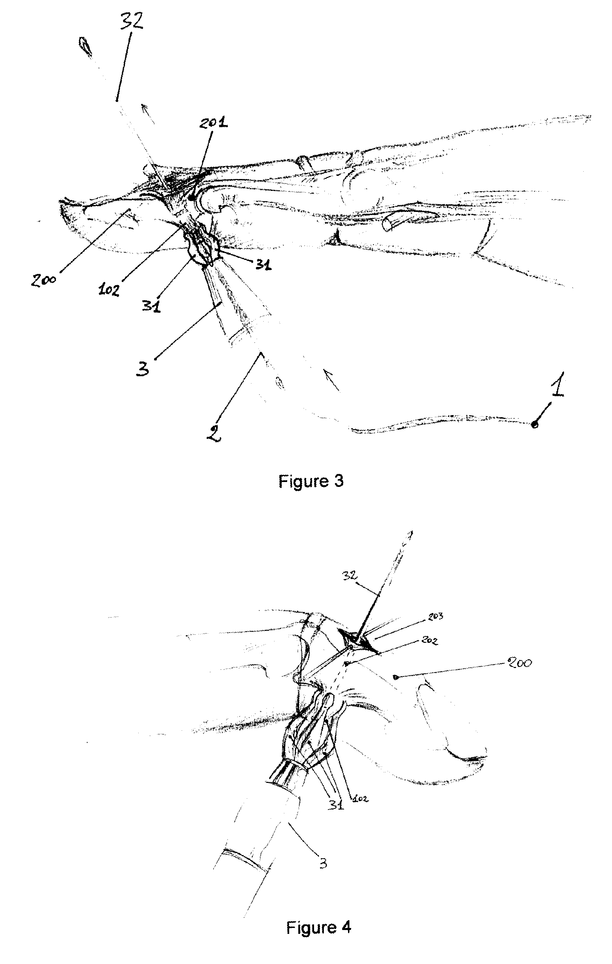

In the second phase of the operation, shown in figures 3-4-5, the surgeon drills a hole, where to insert said specific needle [2] in the base of the phalanx [200], and infibulates said endotendineous thread [1] into the distal lead of the tendon [102] as already seen in figure 1. Therefore, the surgeon requires usage of the holder [3] with angularly spaced jawns [31], to center the distal tendon lead [102] during its longitudinal perforation, made by the coaxial drill piece [32], which is integrated into the said holder instrument [3].

As shown in Figures 3 and 4, the drilling action of said drill piece [32] continues in the base [201] of the distal phalanx [200], creating an endosseus hole [202], which is required for passing the needle [2] and the thread [1].

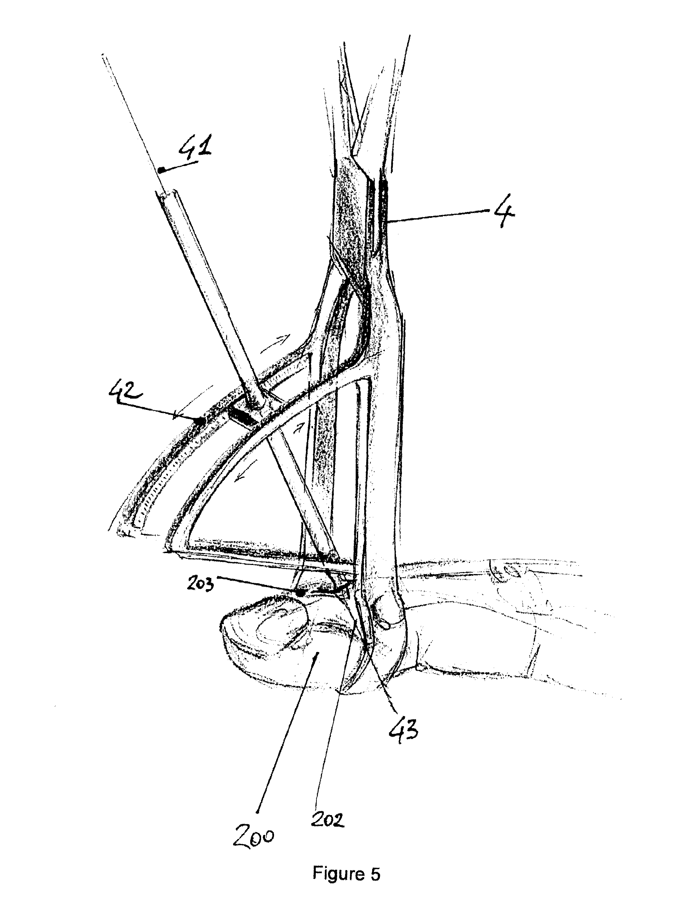

In some cases of injury and, for example, when the lead of the distal tendon [102] is very damaged or nonexistent, the surgeon may evaluate and choose a different alternative way to create said endosseous hole [202] starting the drilling operation from the back [203] of the phalanx. To this purpose, the surgical set includes a specific contouring plier [4], which clamps the distal phalanx [200] and guides the drill piece [41] by means of an adjustable mechanism [42] to set the inclination of the perforation axis [43], as shown in figure 5.

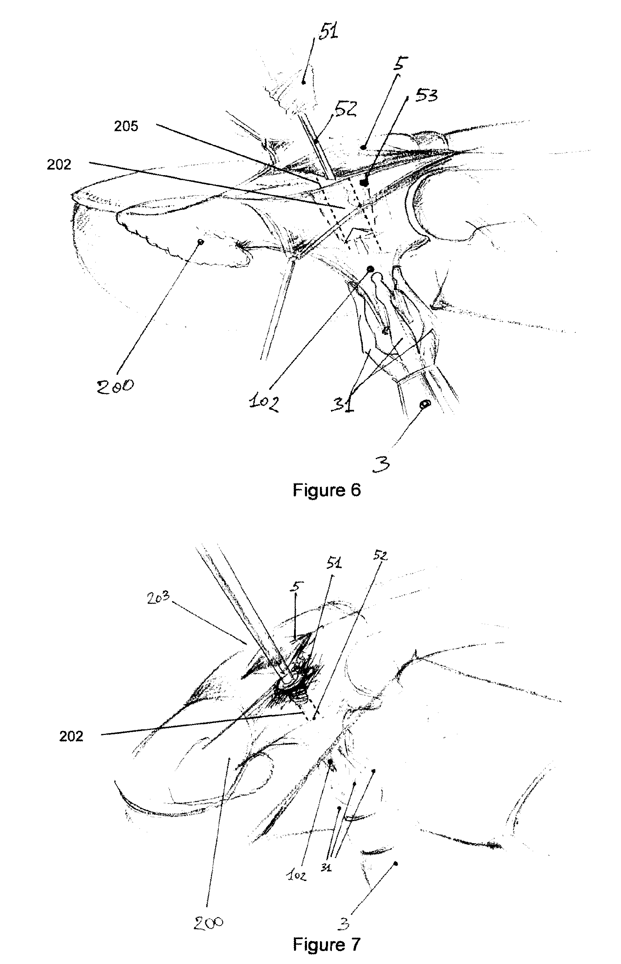

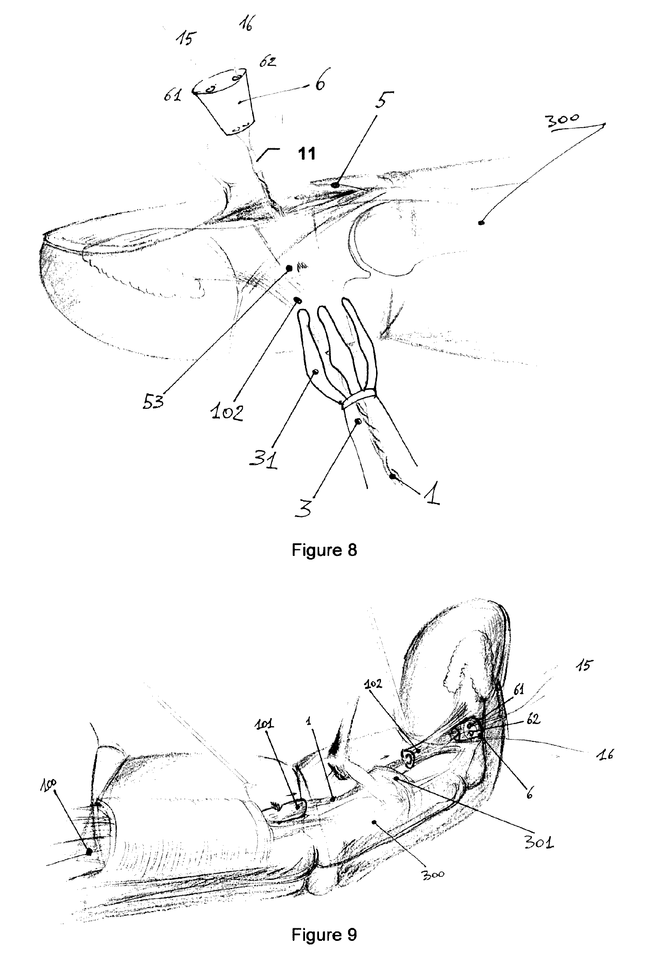

In the third phase of the operation, shown in figures 6-7, the surgeon prepares the back [203] of the phalanx for hosting the anchor [6] of the thread [1]. This tapered anchor piece [6] is made of resorbable biocompatible material high and needs a housing, that is a tapered recess [53], drilled by said tapered mill cutter [51]. Such mill cutter piece [51] is integrated with a guide wire [52] to be inserted into the hole mouth [205] of said endosseous passage [202] previously drilled in the phalanx [200]. Said tapered mill cutter piece [51] is an essential part of the surgical set of instruments, matched to the shape of the tapered anchor piece [6]. In the fourth phase of the operation, as depicted in figures 8-9, the surgeon uses the surgical needle [2] for passing the thread [1] in the distal tendon lead [102] and in the tapered anchor [6], just after having passed the said thread [1] below the pulleys [301] of the articulation [300] and into the endosseous passage[202]. The surgeon adjusts the tension of the thread [1] and lock it after a reasonable functional assessment of the articulation [300].

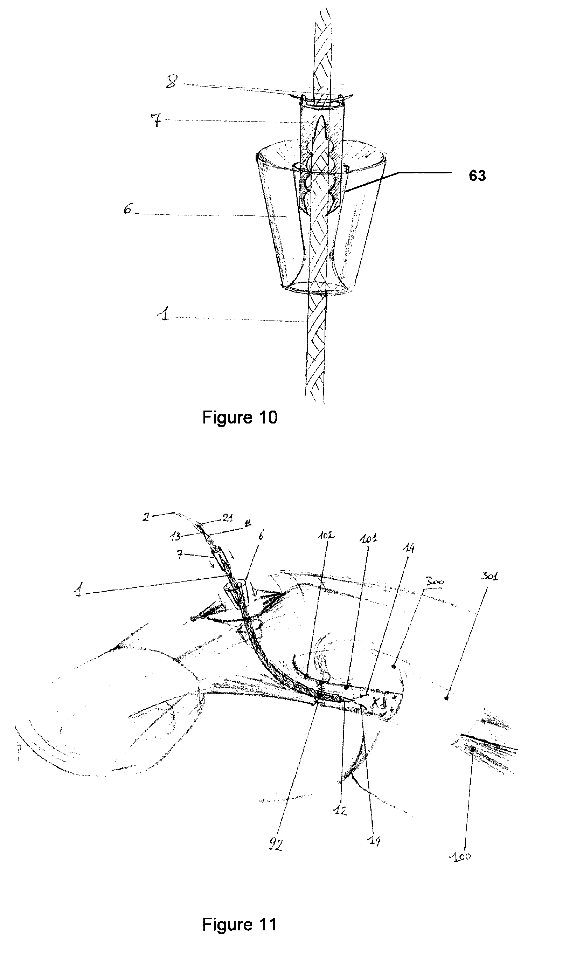

Two possible methods of locking are depicted in figures 8-9 and in figures 10-11 for illustrative purposes and not for limiting the adoption of other methods. Therefore, the surgical set of instruments in subject shall include several different types of tapered anchor pieces [6].

The first method of locking is based on splitting said thread [1] into two leads [15, 16] such that these ends, coming out from the endosseus passage [202], are then passed into appropriate holes [61, 62] of said anchor [6] to be knotted together. The second method is characterized by the tapered tweezer [7] housed in the tapered hole [63] of the anchor [6] in such a way that the thread [1] can move only in one direction, while it is clamped when sliding in the opposite direction. The final locking of said tweezer [7] and of said thread [1] within the anchor [6], eventually, may be secured using a small pin [8].

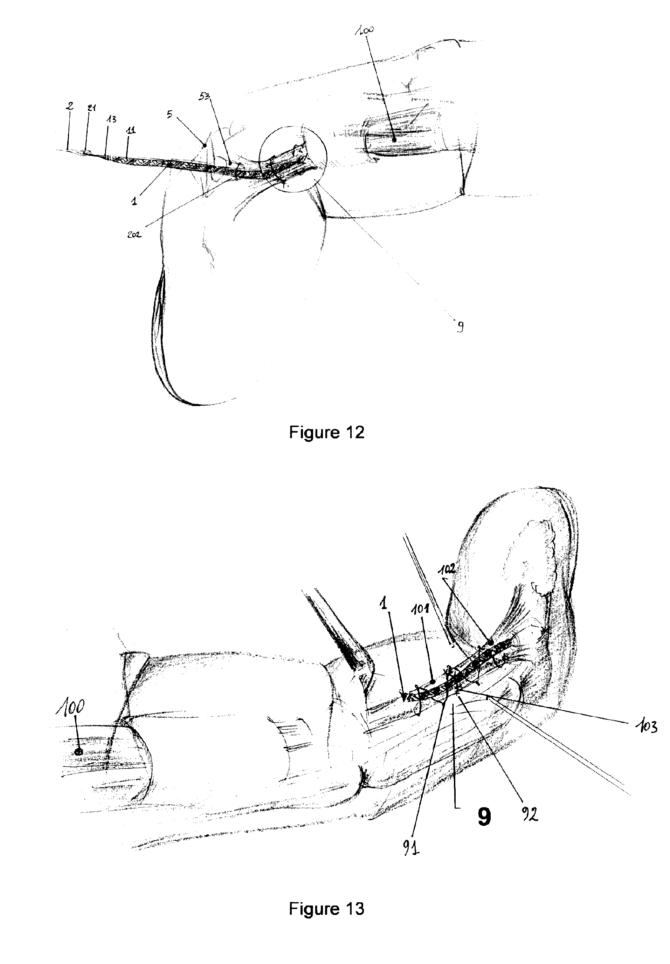

In the last phase of the operation, shown in figures 12-13, the surgeon performs the final suture [9] of the two leads of the tendon [100] using two types of bites. The surgeon places the final suture [9] using two specific types of suture threads, needed for best repairing the broken tendon [100].

After adjusting the tension of the thread [1], the surgeon places the core bites at both ends of the tendon, proximal lead end [101] and distal lead end [102] : these bites [91] pierce the core of said endotendineous thread [1] for achieving the maximum strength of the tendon [100] after its synthesis.

To complete the suture [9], the surgeon places the finishing bites [92], to pull together the edges [103] of the broken tendon leads [101] [102], such that - during the convalescence period - the tendon self-reconstruction is favoured.

Therefore, the surgical set of instruments in subject includes also these threads for sutures [91] and [92], essentially having two different gauges.

## Claims

1. A surgical set of instruments, for repairing the flexor tendons of the hand, essentially composed of the following plurality of surgical instruments : an high strenght endotendineous thread (1) with relevant specific surgical needle (2), an holder instrument (3) integrated with a coaxial drill piece (32), a contouring plier (4) integrated with a drill piece (41) and relevant mechanism (42), a tapered mill cutter piece (51) integrated with its guide wire (52), a tapered anchor (6) optionally housing a small conical tweezer (7) with relevant locking pin (8), some suture threads needed for a core suture (91) and for a finishing suture (92) of the tendon to be repaired.

2. A surgical set of instruments, according to claim 1, wherein the initial section (11) of said high strenght endotendineous thread (1) is equipped with a coupling device (13), hooking the eye (21) of the surgical needle (2), while the final section (12) of the thread itself (1) ends with some small threads (14) suitable for suturing and for reinforcing the proximal end (101) of the broken tendon to be repaired (100).

3. A surgical set of instruments, according to claim 1, wherein said holder instrument (3) is made of angularly spaced jaws (31), suitables for holding the distal end (102) of the tendon to be repaired, and - moreover - such holder (3) includes a coaxial drill piece (32) for longitudinally piercing the core of the distal tendon end (102) and for piercing the bone at the base (201) of the distal phalanx (200).

4. A surgical set of instruments, according to claim 1 , wherein said contouring plier (4) is made suitably shaped to firmly clamp the distal phalanx (200) and-moreover - such contouring plier (4) leads an integrated drill piece (41) on the back (203) of the phalanx (200) in order to pierce the hole (202) according to an adjustable tilt axis (43) set by the proper mechanism (42), belonging to the contouring plier (4) itself.

5. A surgical set of instruments, according to claim 1, wherein said tapered mill cutter piece (51) is provided with a guide wire (52) to be inserted into the hole (205) in order to create a tapered recess (53) into the bone on the back (203) of the phalanx (200).

6. A surgical set of instruments, according to claim 1, wherein said tapered anchor (6) is made of bio-compatible material and is shaped to allow the insertion of the initial section (11) of the high strenght endotendineous thread (1), trapping it after sliding it and properly adjusting its tension.

7. A surgical set of instruments, according to the previous claims 1 and 6, wherein said tapered anchor (6) is provided with two holes (61 and 62) passing the two threads (15) and (16) resulting from splitting the initial section (11) of said thread (1), such that they are pulled together for properly adjusting their tension and, finally, they are knotted together to ensure locking of said thread (1) and stability over time.

8. A surgical set of instruments, according to claims 1 and 6, wherein said tapered anchor (6), made as a different realization than the former one of claim 7, is provided with a conical hole (63), which is housing the conical tweezer (7) for sliding the high strenght thread (1) and for properly trapping it, as needed.

9. A surgical set of instruments, according to claims 1, 6 and 8, wherein, the pin (8) is inserted inside the conical tweezer (7) for locking the whole assembly of said high strenght thread (1), firmly trapped into the said anchor (6).

10. A surgical set of instruments, according to claim 1, wherein suture threads of two different gauges are essentially provided, for placing core suture bites (91) through the proximal end (101) and the distal end (102) of tendon core, as reinforced by the high strenght endotendineous thread (1), and for further placing a finishing suture (92) made of small and superficial bites, pulling together the tendon edges (103).
